# EUROPÄISCHE PATENTSCHRIFT

(11) **EP 0 568 574 B1**
(45) Veröffentlichungstag und Bekanntmachung des Hinweises auf die Patenterteilung: **09.04.1997**
(21) Anmeldenummer: 92903293.6
(22) Anmeldetag: 21.01.1992
(51) Int. Cl.: C08B 37/00, A61K 49/00

(54) **VERFAHREN ZUR HERSTELLUNG EINES PYROGENFREIEN GUT WASSERLÖSLICHEN FRUCTANS SOWIE EIN NIERENDIAGNOSTIKUM, DAS EIN SOLCHES FRUCTAN ENTHÄLT**
METHOD OF PREPARATION OF A PYROGEN-FREE FRUCTANE WHICH IS READILY SOLUBLE IN WATER, AND AN AGENT CONTAINING SUCH A FRUCTANE FOR THE EXAMINATION OF KIDNEY FUNCTION
PROCEDE POUR LA FABRICATION D'UN FRUCTANE SANS PYROGENE ET BIEN SOLUBLE DANS L'EAU, AINSI QU'UN AGENT DIAGNOSTIQUE RENAL CONTENANT UN TEL FRUCTANE

(30) Priorität: 23.01.1991 DE 4101910
(43) Veröffentlichungstag der Anmeldung: 10.11.1993
(73) Patentinhaber: Laevosan-Gesellschaft m.b.H., A-4020 Linz (AT)
(72) Erfinder: NITSCH, Ernst, A-4040 Linz (AT)
(74) Vertreter: Huber, Bernhard, Dipl.-Chem.
(86) Internationale Anmeldenummer: EP9200120
(87) Internationale Veröffentlichungsnummer: WO9213005

(56) Entgegenhaltungen:
- FR-A- 2 117 917
- WORLD PATENTS INDEX LATEST Section Ch, Week 9004, Derwent Publications Ltd., London, GB; Class B, AN 90-026249
- WORLD PATENTS INDEX Section Ch, Week 7635, Derwent Publications Ltd., London, GB; Class B, AN 76-66213X
- WORLD PATENTS INDEX Section Ch, Week 8016, Derwent Publications Ltd., London, GB; Class B, AN 80-28084C

## Beschreibung

Die Erfindung betrifft ein Verfahren zur Herstellung eines pyrogenfreien gut wasserlöslichen Fructans sowie ein parenteral applizierbares Nierendiagnostikum auf der Basis eines solchen Fructans.

Fructane, auch Polyfructosane genannt, sind Oligo- und Polysaccharide, die aus auf ein Grundmolekül Saccharose aufgepfropften geraden oder verzweigten Fructoseketten aufgebaut sind. Je nach Verzweigungs- und Polymerisationsgrad können wesentliche Unterschiede bei ihren physikalischen Eigenschaften, wie z.B. der Wasserlöslichkeit, auftreten. Viele Fructane werden in Pflanzen als Reservekohlehydrate gespeichert, und kommen vor allem in den unterirdischen Teilen von Compositen, Campanulaceen, Gräsern und Liliaceen vor.

Eine besondere Verwendung finden Fructane in der Nierendiagnostik, insbesondere zur Bestimmung der glomuleren Filtrationsrate. Üblicherweise wird dazu Inulin, ein Fructan aus den Wurzeln verschiedener Compositenarten, wie Zichorien, Dahlien und Topinambur, als Standardtestsubstanz eingesetzt (A.N. Richard, B.B. Westfall, P.A. Bott: Proc.Soc.exp.Biol., N.Y. 32 (1934), 73 und 0. Schück: Examination of Kidney Function, Martinus Nijnhoff Publishers, Boston 1984, S. 9 ff). Inulin weist den Vorteil auf, daß es nach parenteraler Applikation weder durch den Stoffwechsel verändert noch im Organismus gespeichert wird, sondern durch die Nierenglomeruli ausfiltriert und in den Tubuli nicht wieder rückresorbiert wird. (J.A. Shannon, H.W. Smith: J.clin.Inv. 14 (1935), 393). Inulin weist jedoch für die klinische Alltagsroutine den Nachteil auf, daß es in Wasser nur sehr gering löslich ist und daß daher wäßrige Zubereitungen bei der Lagerung auskristallisieren und vor Applikation durch längeres Erhitzen erneut in Lösung gebracht werden müssen. Durch diese Maßnahme wird jedoch Inulin je nach Dauer der Hitzeeinwirkung hydrolytisch angegriffen und ggfs. bis zur Fructose abgebaut. Ein weiterer Nachteil besteht darin, daß bei unvollständiger Auflösung Reste von nicht gelösten Inulinteilchen in der Zubereitung verbleiben, die nicht ohne weiteres erkennbar sind und die nach einer Injektion schwere Kreislaufkomplikationen hervorrufen können. In der österreichischen Patentschrift 304769 wird daher vorgeschlagen, das Inulin durch andere wasserlösliche Fructane, und zwar aus den Speicherorganen von Pflanzen aus den Familien der Liliaceen, Amaryllidaceen und Gramineen wie Sinistrin und Phlein, als Ersatz für Inulin zu verwenden. Diese sind, ähnlich wie Inulin, auf ca. 10 bis 40 Fructoseeinheiten aufgebaut, entsprechend Molekulargewichten von ca. 1600 bis 6500.

In der älteren Literatur O. Schmiedeberg: Hoppe Seyler's Z.physiol.Chem. 3 (1979), 112 und G. Klein: Handbuch der Pflanzenanalyse, Springer 1932, 1. Teil, S. 866-873, werden diese Fructane aus wäßrigen Extrakten der geeigneten Pflanzenorgane nach Reinigung mit Fällungsmitteln wie z.B. Bleisalzen, durch nachfolgende mehrfach wiederholte Umfällung mit organischen Lösungmitteln wie Methanol, Ethanol oder Aceton gewonnen. Die oben zitierte österreichische Patentschrift ersetzt die Verwendung der giftigen Schwermetallsalze durch eine Fällung mit den genannten Lösungsmitteln im sauren Milieu bei pH-Werten von 1,0 bis 3,0. Hierbei muß mit partiellem Abbau der leicht hydrolysierbaren Fructanketten, Erniedrigung des mittleren Molekulargewichtes und Verlusten an Ausbeute gerechnet werden. Darüberhinaus sind bei allen bisher bekannten Verfahren aufwendige Sicherheitsmaßnahmen wegen Brennbarkeit und Explosionsgefahr erforderlich und es muß mit unerwünschten Lösungsmittelrückständen im Produkt gerechnet werden.

Die Erfindung hat daher zum Ziel ein Verfahren zur Gewinnung von Fructanen bereitzustellen, bei dem keine organischen Lösungsmittel oder giftige Schwermetallsalze verwendet werden müssen und eine Verbesserung der Ausbeute erreicht wird. Darüberhinaus soll das Verfahren auch eine Abtrennung von Pyrogenen sicherstellen, so daß auch kontaminiertes und infiziertes Rohmaterial verwendet werden kann.

Dieses Ziel wird erfindungsgemäß durch ein Verfahren zur Herstellung eines gut wasserlöslichen und pyrogenfreien Fructans erreicht, das dadurch gekennzeichnet ist, daß man einen wäßrigen Extrakt, der auf an sich bekannte Weise aus fructanhaltigen Pflanzenteilen gewonnen wird, einer Ultrafiltration an einer ersten Membran unterzieht, die höhermolekulare Kohlehydrate und Proteine, Pyrogene sowie ggfs. andere Extraktstoffe zurückhält, das Fructan aber noch durchläßt. Mit dem auf diese Weise gewonnenen Ulftrafiltrat wird nun eine zweite Ultrafiltration an einer Membran durchgeführt, die aber nun das Fructan zurückhält, aber Salze, Monosaccharide und niedrige Oligosaccharide durchläßt. Das auf diese Weise gewonnene Fructan ist frei von Pyrogenen und von organischen Lösungsmitteln. Das Retentat der erfindunggemäßen zweiten Ultrafiltration, in dem das Fructan enthalten ist, kann direkt zum Nierendiagnostikum weiterverarbeitet werden oder auch auf an sich bekannte Weise weiter gereinigt und isoliert werden. Dies wird zweckmäßigerweise durch Auftrocknung der Lösung mit oder ohne vorhergehende Reinigung an Ionenaustauschern durchgeführt.

Vorzugsweise werden im erfindungsgemäßen Verfahren für die erste Ultrafiltration solche Membranen verwendet, die eine Ausschlußgrenze von 30.000 bis 100.000 Dalton, insbesondere von 30.000 bis 50.000 Dalton aufweisen. Durch solche Membranen werden Proteine, Pyrogene und höhermolekulare Polysaccharide wirksam zurückgehalten. Das Filtrat enthält das extrahierte Fructan zusammen mit niedermolekularen Begleitstoffen vollständig. Für die zweite Ultrafiltration werden erfindungsgemäß vorzugsweise solche Membranen verwendet, die eine Ausschlußgrenze von 800 bis 2000 Dalton, insbesondere von 1000 bis 1500 Dalton aufweisen. Durch solche Membranen wird das zu isolierende Fructan zurückgehalten, jedoch unerwünschte niedermolekulare Substanzen wie Salze, mono- und oligomere Kohlenhydrate mit dem Filtrat entfernt. Das weitgehend gereinigte Fructan liegt dann ggfs. nach einer Diafiltration unter Nachspeisen von Wasser im Retentat in guter Ausbeute vor. Das Produkt kann anschließend noch mit einem zusätzlichen Entfärbungs- und/oder Entsalzungsschritt auf an sich bekannte Weise weiter gereinigt werden.

Geeignete Ultrafiltrationsmembranen sind z.B. für die erste Membran Rohrmembran FP 100 der Firma Paterson, Candy, England, und für die zweite Membran Spiralmembranen Desal GE 4026F der Firma Desalination Systems, Calif., USA.

Die so gewonnene reine Fructanlösung kann nun direkt auf die gewünschte Konzentration eingestellt und in sterilisierbare Behältnisse wie Ampullen und Infusionsflaschen gefüllt oder auf schonende Weise getrocknet und für den späteren Gebrauch aufbewahrt werden. Als schonende Trocknungsweise eignet sich für das erfindungsgemäße Verfahren die Lyophilisation, Vakuum- oder Sprühtrocknung.

Die Erfindung betrifft auch ein Nierendiagnostikum, das zumindest ein erfindungsgemäß hergestelltes Fructan zusammen mit einer osmotisch wirkenden physiologisch verträglichen Puffersubstanz enthält. Der pH des Puffers liegt zwischen 5,0 und 7,0 und vorzugsweise zwischen 6,0 und 6,5 und ist in einer solchen Menge im Nierendiagnostikum enthalten, daß eine der Blutisotonie entsprechende Osmolalität von 250 bis 350, insbesondere zwischen 280 und 320 mOsmol/kg H₂O erreicht wird.

Als physiologisch verträgliche Puffersubstanzen werden erfindungsgemäß Gemische von schwachen Säuren, insbesondere schwachen organischen Säuren, sowie Alkali- oder Erdalkalisalzen davon verwendet. Bevorzugte Säuren sind Essigsäure und Milchsäure und deren Natriumsalze. Erfindungsgemäß ist es auch möglich, als verträgliche Puffersubstanzen Gemische schwacher Basen, insbesondere organischer Basen, und deren Salze, vorzugsweise deren Hydrochloridsalze, zu verwenden. Bevorzugte Basen sind Triethanolamin und basische Aminosäuren.

Das erfindungsgemäße Nierendiagnostikum weist im Gegensatz zu den Präparaten des Standes der Technik, die üblicherweise aus sterilisierten wäßrigen Lösungen von Inulin bzw. wasserlöslichen Fructanen ohne weitere Zusätze (z.B. 10 g Inulin pro 100 ml oder 25 g Sinistrin pro 100 ml) bestehen, eine außergewöhnlich gute Verträglichkeit und Haltbarkeit auf.

Bei den Nierendiagnostika des Standes der Technik entsteht bei rascher Applikation des Präparates die Gefahr von Schmerzen und Hämolysen. Außerdem hat es sich gezeigt, daß bei solchen Systemen während der Hitzesterilisation und bei längerer Lagerung in warmer Umgebung eine Abnahme des pH-Wertes eintritt, die zu einem starken Abbau der Fructane führt.

Die Erfindung wird durch die folgenden Beispiele näher erläutert.

### Beispiel 1

### Herstellung des Fructans aus Knoblauch (Sinistrin)

10 kg handelsübliches trockenes Knoblauchgranulat wurden mit einer Lösung von 10 g Natriumsulfit in 30 l entsalztem wasser gleichmäßig angerührt und für 24 h in einem geschlossenen Behälter der Quellung überlassen. Die Masse wurde in einem Perkolator mit entsalztem Wasser so lange bei einem Fluß von 4 l/h extrahiert, bis 100 l Filtrat vorlagen. Dieses wurde über ein Ultrafiltrations-Wickelmodul 1 mit einer Ausschlußgrenze von 50.000 Dalton filtriert und das Filtrat über ein Zwischengefäß sofort wieder auf ein Ultrafiltrations-Wickelmodul 2 mit einer Ausschlußgrenze von 1000 Dalton aufgegeben. Nachdem der ganze Extrakt auf Modul 1 aufgegeben war, wurde mit ca. 5 l Wasser nachgewaschen und das Retentat von Modul 1 verworfen. Das Retentat von Modul 2 wurde unter laufendem Ersatz des Filtratvolumens durch entsalztes Wasser so lange der Diafiltration unterworfen, bis die Leitfähigkeit im Filtrat kleiner als 200 µS/cm war. Alle Filtrate von Modul 2, welche Salze, mono- und oligomere Kohlenhydrate und andere niedermolekulare Substanzen enthalten, wurden verworfen. Das Retentat bestand aus 55 kg einer bereits sehr reinen Lösung mit 7,4 % Fructan, entsprechend 4,07 kg Trockenmasse. Zur weiteren Reinigung von Spuren von Geruchs- und Farbstoffen wurden 50 g Aktivkohle eingerührt und nach einer Einwirkzeit von 1 h zur Abtrennung der Kohle über Filterschichten unter Nachwaschen filtriert. Das farb- und geruchlose Filtrat wurde im Vakuum zur Sirupkonsistenz eingeengt und in Wannen im Vakuumtrockenschrank bei 80°C zu einer weißen, blasigen Masse getrocknet.

Ausbeute nach Mahlen und Abfüllen 3,95 kg
Spezifische Drehung /alpha/20/D = -39,0° (c = 10; H₂O) Molekulargewicht Mw (Gelpermeationschromatographie) = 3650 Bei der Testung am Kaninchen nach Pharm.Eur. erwies sich die Substanz als pyrogenfrei.

### Beispiel 2

### Herstellung des Fructans aus roter Meerzwiebel (Sinistrin)

10 kg handelsüblicher getrockneter roter Meerzwiebeln wurden grob gemahlen und Extraktion sowie doppelte Ultrafiltration wie in Beispiel 1 durchgeführt. Das Retentat bestand aus 49 kg einer schwach rotgefärbten Lösung mit 9,0 % Fructan, entsprechend 4,41 kg Trockenmasse.

Zur weiteren Reinigung wurde die Lösung über ein Mischbett-Austauschersystem aus je 200 ml stark saurem Kationenaustauscher in der H⁺-Form und mittelstark basischem Anionenaustauscher in der OH⁻-Form gegeben. Die nun vollkommen farblose Lösung wurde nach Einengen im Vakuum wie in Beispiel 1 getrocknet.

Ausbeute nach Mahlen und Abfüllen 4,2 kg.
Spezifische Drehung /alpha/20/D = - 39,5° (c = 10; H₂O) Molekulargewicht Mw (Gelpermeationschromatographie) = 3820 Bei der Testung am Kaninchen nach Pharm.Eur. erwies sich die Substanz als pyrogenfrei.

### Beispiel 3

### Herstellung eines isotonen lactatgepufferten Nierendiagnostikums zur parenteralen Anwendung

13,4 g Natriumlactatlösung 50 % DAB 9 wurden mit 700 ml dest. Wasser zur Injektion gemischt, 250 g des Fructans aus Knoblauch, gewonnen nach Beispiel 1 bis zur vollständigen Auflösung eingerührt und durch Zugabe von 6,8 ml 0,1 n Natronlauge der pH-Wert auf 6,30 eingestellt.

Zuletzt wurde mit dest. Wasser auf 1 l ergänzt.

Die Lösung wurde zur Entkeimung über ein 0,2 µ Membranfilter filtriert und zu 20 ml in Ampullen abgefüllt, welche verschlossen und 8 Min. bei 121°C sterilisiert wurden. Es resultierte eine farblose, pyrogenfreie, sterile Lösung, welche bei intravenöser Injektion an Mensch und Tier reaktions- und schmerzlos vertragen wurde und allen Qualitätsanforderungen entsprach.

Osmolalität (Gefrierpunktsmethode): 311 mOsmol/kg H₂O

### Beispiel 4:

### Herstellung eines isotonen acetatgepufferten Nierendiagnostikums zur parenteralen Anwendung

8,0 g Natriumacetat-Trihydrat wurden mit 700 ml dest. Wasser zur Injektion gemischt, 250 g des Fructans aus Meerzwiebel, gewonnen nach Beispiel 2, bis zur vollständigen Auflösung eingerührt und durch Zugabe von 2,0 ml 1 m Natronlauge der pH-Wert auf 6,30 eingestellt.

Zuletzt wurde mit dest. Wasser auf 1 l ergänzt.

Die Lösung wurde zur Entkeimung über ein 0,2 µ Membranfilter filtriert und zu 20 ml in Ampullen abgefüllt, welche verschlossen und 8 Min. bei 121°C sterilisiert wurden. Es resultierte eine farblose, pyrogenfreie, sterile Lösung, welche bei intravenöser Injektion an Mensch und Tier reaktions- und schmerzlos vertragen wurde und allen Qualitätsanforderungen entsprach.

Osmolalität (Gefrierpunktsmethode): 303 mOsmol/kg H₂O

## Patentansprüche (Patentansprüche für folgende(n) Vertragsstaat(en): AT, BE, CH, LI, DE, DK, FR, GB, GR, IT, LU, NL, SE)

1. Parenteral applizierbares Nierendiagnostikum,
**dadurch gekennzeichnet,**
daß es ein pyrogenfreies, gut wasserlösliches Fructan, erhältlich durch Extrahieren von fructanhaltigen Pflanzenteilen mit Wasser in an sich bekannter Weise, Ultrafiltrieren des wäßrigen Extrakts an einer ersten Membran, die höhermolekulare Kohlehydrate und Proteine zurückhält, das Fructan aber durchläßt und Ultrafiltrieren des Filtrats der ersten Ultrafiltration an einer zweiten Membran, die das Fructan zurückhält, aber Salze, Monosaccharide und niedrige Oligosaccharide durchläßt, und ein physiologisch unbedenkliches Puffersystem mit einem pH von 5,0 bis 7,0 in einer solchen Menge enthält, daß die fertige Lösung blutisotonisch ist.

2. Parenteral applizierbares Nierendiagnostikum nach Anspruch 1,
**dadurch gekennzeichnet,**
daß es einen pH von 6,0 bis 6,5 aufweist.

3. Nierendiagnostikum nach Anspruch 1 oder 2,
**dadurch gekennzeichnet,**
daß das Puffersystem aus einer physiologisch unbedenklichen organischen Säure und deren Alkali- und/oder Erdalkalisalz besteht.

4. Nierendiagnostikum nach einem der Ansprüche 1 bis 3,
**dadurch gekennzeichnet,**
daß es als Puffersystem Milchsäure und Natriumlactat enthält.

5. Nierendiagnostikum nach einem der Ansprüche 1 bis 3,
**dadurch gekennzeichnet,**
daß es als Puffersystem Essigsäure und Natriumacetat enthält.

6. Nierendiagnostikum nach einem der Ansprüche 1 und 2,
**dadurch gekennzeichnet,**
daß es als Puffersystem eine Mischung aus einer physiologisch unbedenklichen organischen Base und deren Salz(en) enthält.

7. Nierendiagnostikum nach Anspruch 6,
**dadurch gekennzeichnet,**
daß das Puffersystem Triethanolamin und dessen Hydrochlorid ist.

8. Nierendiagnostikum nach einem der Ansprüche 1, 2 und 6,
**dadurch gekennzeichnet,**
daß das Puffersystem eine basische Aminosäure und deren Hydrochlorid ist.

9. Nierendiagnostikum nach Anspruch 8,
**dadurch gekennzeichnet,**
daß die basische Aminosäure Lysin und/oder Ornithin ist.

10. Nierendiagnostikum nach einem der Ansprüche 1 bis 9,
**dadurch gekennzeichnet,**
daß es das Puffersystem in einer solchen Konzentration enthält, daß die fertige Lösung eine Osmolalität von 250 bis 350 mOsmol/kg H₂O aufweist.

11. Nierendiagnostikum nach Anspruch 10,
**dadurch gekennzeichnet,**
daß die Osmolalität 280 bis 320 mOsmol/kg H₂O beträgt.

12. Nierendiagnostikum nach einem der Ansprüche 1 bis 11,
**dadurch gekennzeichnet,**
daß es ein Fructan aus Liliazeen, Amaryllidazeen oder/und Gramineen enthält.

13. Nierendiagnostikum nach einem der Ansprüche 1 bis 12,
**dadurch gekennzeichnet,**
daß es als Fructan Sinistrin enthält.

## Patentansprüche (Patentansprüche für folgende(n) Vertragsstaat(en): ES)

1. Verfahren zur Herstellung eines parenteral applizierbaren Nierendiagnostikums,
**dadurch gekennzeichnet,**
daß ein pyrogenfreies, gut wasserlösliches Fructan durch Extrahieren von fructanhaltigen Pflanzenteilen mit Wasser in an sich bekannter Weise, Ultrafiltrieren des wäßrigen Extrakts an einer ersten Membran, die höhermolekulare Kohlehydrate und Proteine zurückhält, das Fructan aber durchläßt, und Ultrafiltrieren des Filtrats der ersten Ultrafiltration an einer zweiten Membran, die das Fructan zurückhält, aber Salze, Monosaccharide und niedrige Oligosaccharide durchläßt, hergestellt und der so erhaltenen Fructanlösung ein physiologisch unbedenkliches Puffersystem mit einem pH von 5,0 bis 7,0 in einer solchen Menge, daß die fertige Lösung blutisotonisch ist, zugesetzt wird.

2. Verfahren nach Anspruch 1,
**dadurch gekennzeichnet,**
daß man auf einen pH von 6,0 bis 6,5 einstellt.

3. Verfahren nach Anspruch 1 oder 2,
**dadurch gekennzeichnet,**
daß das Puffersystem aus einer physiologisch unbedenklichen organischen Säure und deren Alkali- und/oder Erdalkalisalz besteht.

4. Verfahren nach einem der Ansprüche 1 bis 3,
**dadurch gekennzeichnet,**
daß man als Puffersystem Milchsäure und Natriumlactat verwendet.

5. Verfahren nach einem der Ansprüche 1 bis 3,
**dadurch gekennzeichnet,**
daß man als Puffersystem Essigsäure und Natriumacetat verwendet.

6. Verfahren nach einem der Ansprüche 1 und 2,
**dadurch gekennzeichnet,**
daß man als Puffersystem eine Mischung aus einer physiologisch unbedenklichen organischen Base und deren Salz(en) verwendet.

7. Verfahren nach Anspruch 6,
**dadurch gekennzeichnet,**
daß das Puffersystem Triethanolamin und dessen Hydrochlorid ist.

8. Verfahren nach einem der Ansprüche 1, 2 und 6,
**dadurch gekennzeichnet,**
daß das Puffersystem eine basische Aminosäure und deren Hydrochlorid ist.

9. Verfahren nach Anspruch 8,
**dadurch gekennzeichnet,**
daß die basische Aminosäure Lysin und/oder Ornithin ist.

10. Verfahren nach einem der Ansprüche 1 bis 9,
**dadurch gekennzeichnet,**
daß das Puffersystem in einer solchen Konzentration verwendet wird, daß die fertige Lösung eine Osmolalität von 250 bis 350 mOsmol/kg H₂O aufweist.

11. Verfahren nach Anspruch 10,
**dadurch gekennzeichnet,**
daß die Osmolalität 280 bis 320 mOsmol/kg H₂O beträgt.

12. Verfahren nach einem der Ansprüche 1 bis 11,
**dadurch gekennzeichnet,**
daß ein Fructan aus Liliazeen, Amaryllidazeen oder/und Gramineen verwendet wird.

13. Verfahren nach einem der Ansprüche 1 bis 12,
**dadurch gekennzeichnet,**
daß als Fructan Sinistrin verwendet wird.

## Claims (Claims for the following Contracting State(s): AT, BE, CH, LI, DE, DK, FR, GB, GR, IT, LU, NL, SE)

1. Renal diagnostic agent which can be applied parenterally,
**wherein**
it contains a pyrogen-free, readily water soluble fructan, obtainable by extracting parts of plants containing fructan with water, ultrafiltering the aqueous extract on a first membrane which retains higher-molecular carbohydrates and proteins but allows passage of fructan and ultrafiltering the filtrate from the first ultrafiltration on a second membrane which retains fructan but allows passage of salts, monosaccharides and low oligosaccharides, and contains a physiologically acceptable buffer system with a pH of 5.0 to 7.0 in such an amount that the final solution has the same osmotic pressure as blood.

2. Renal diagnostic agent which can be applied parenterally as claimed in claim 1,
**wherein**
it has a pH of 6.0 to 6.5.

3. Renal diagnostic agent as claimed in claim 1 or 2,
**wherein**
the buffer system consists of a physiologically acceptable organic acid and its alkali and/or alkaline earth salt.

4. Renal diagnostic agent as claimed in one of the claims 1 to 3,
**wherein**
it contains lactic acid and sodium lactate as the buffer system.

5. Renal diagnostic agent as claimed in one of the claims 1 to 3,
**wherein**
it contains acetic acid and sodium acetate as the buffer system.

6. Renal diagnostic agent as claimed in one of the claims 1 and 2,
**wherein**
it contains amixture of a physiologically acceptable organic base and its salt(s) as the buffer system.

7. Renal diagnostic agent as claimed in claim 6,
**wherein**
the buffer system is triethanolamine and its hydrochloride.

8. Renal diagnostic agent as claimed in one of the claims 1, 2 and 6,
**wherein**
the buffer system is a basic amino acid and its hydrochloride.

9. Renal diagnostic agent as claimed in claim 8,
**wherein**
the basic amino acid is lysine and/or ornithine.

10. Renal diagnostic agent as claimed in one of the claims 1 to 9,
**wherein**
it contains the buffer system at such a concentration that the final solution has an osmolality of 250 to 350 mOsmol/kg H₂O.

11. Renal diagnostic agent as claimed in claim 10,
**wherein**
the osmolality is 280 to 320 mOsmol/kg H₂O.

12. Renal diagnostic agent as claimed in one of the claims 1 to 11,
**wherein**
it contains a fructan from Liliaceae, Amaryllidaceae or/and Gramineae.

13. Renal diagnostic agent as claimed in one of the claims 1 to 12,
**wherein**
it contains sinistrin as the fructan.

## Claims (Claims for the following Contracting State(s): ES)

1. Process for the production of a renal diagnostic agent which can be applied parenterally,
**wherein**
it contains a pyrogen-free, readily water soluble fructan, obtainable by extracting parts of plants containing fructan with water, ultrafiltering the aqueous extract on a first membrane which retains higher-molecular carbohydrates and proteins but allows passage of fructan and ultrafiltering the filtrate from the first ultrafiltration on a second membrane which retains fructan but allows passage of salts, monosaccharides and low oligosaccharides, and contains a physiologically acceptable buffer system with a pH of 5.0 to 7.0 in such an amount that the final solution has the same osmotic pressure as blood.

2. Process as claimed in claim 1,
wherein
it is adjusted to a pH of 6.0 to 6.5.

3. Process as claimed in claim 1 or 2,
**wherein**
the buffer system consists of a physiologically acceptable organic acid and its alkali and/or alkaline earth salt.

4. Process as claimed in one of the claims 1 to 3,
**wherein**
lactic acid and sodium lactate are used as the buffer system.

5. Process as claimed in one of the claims 1 to 3,
**wherein**
acetic acid and sodium acetate are used as the buffer system.

6. Process as claimed in one of the claims 1 and 2,
**wherein**
a mixture of a physiologically acceptable organic base and its salt(s) are used as the buffer system.

7. Process as claimed in claim 6,
**wherein**
the buffer system is triethanolamine and its hydrochloride.

8. Process as claimed in one of the claims 1, 2 and 6,
**wherein**
the buffer system is a basic amino acid and its hydrochloride.

9. Process as claimed in claim 8,
**wherein**
the basic amino acid is lysine and/or ornithine.

10. Process as claimed in one of the claims 1 to 9,
**wherein**
the buffer system is used at such a concentration that the final solution has an osmolality of 250 to 350 mOsmol/kg H₂O.

11. Process as claimed in claim 10,
**wherein**
the osmolality is 280 to 320 mOsmol/kg H₂O.

12. Process as claimed in one of the claims 1 to 11,
**wherein**
a fructan from Liliaceae, Amaryllidaceae or/and Gramineae is used.

13. Process as claimed in one of the claims 1 to 12,
**wherein**
sinistrin is used as the fructan.

## Revendications (Revendications pour l'(les) Etat(s) contractant(s) suivant(s): AT, BE, CH, LI, DE, DK, FR, GB, GR, IT, LU, NL, SE)

1. Agent diagnostique de la fonction rénale pouvant être administré par voie parentérale, caractérisé en ce qu'il contient un fructane sans pyrogène, bien soluble dans l'eau, pouvant être obtenu par extraction de façon connue en soi, avec de l'eau, à partir d'éléments de plantes contenant du fructane, ultrafiltration de l'extrait aqueux sur une première membrane qui retient les hydrates de carbone et les protéines de masse molaire élevée mais qui laisse passer le fructane, et ultrafiltration du filtrat de la première ultrafiltration sur une seconde membrane qui retient le fructane, mais qui laisse passer les sels, les monosaccharides et les oligosaccharides inférieurs, et un système tampon physiologiquement acceptable ayant un pH de 5,0 à 7,0 en une quantité telle que la solution finale est isotonique au sang.

2. Agent diagnostique de la fonction rénale pouvant être administré par voie parentérale selon la revendication 1, caractérisé en ce qu'il présente un pH de 6,0 à 6,5.

3. Agent diagnostique de la fonction rénale selon la revendication 1 ou 2, caractérisé en ce que le système tampon est constitué d'un acide organique physiologiquement acceptable et d'un de ses sels de métaux alcalins et/ou de métaux alcalino-terreux.

4. Agent diagnostique de la fonction rénale selon l'une des revendications 1 à 3, caractérisé en ce qu'il contient comme système tampon de l'acide lactique et du lactate de sodium.

5. Agent diagnostique de la fonction rénale selon l'une des revendications 1 à 3, caractérisé en ce qu'il contient comme système tampon de l'acide acétique et de l'acétate de sodium.

6. Agent diagnostique de la fonction rénale selon l'une des revendications 1 et 2, caractérisé en ce qu'il contient comme système tampon un mélange d'une base organique physiologiquement acceptable et d'un ou plusieurs de ses sels.

7. Agent diagnostique de la fonction rénale selon la revendication 6, caractérisé en ce que le système tampon est constitué par la triéthanolamine et son chlorhydrate.

8. Agent diagnostique de la fonction rénale selon l'une des revendications 1, 2 et 6, caractérisé en ce que le système tampon est constitué par un aminoacide basique et son chlorhydrate.

9. Agent diagnostique de la fonction rénale selon la revendication 8, caractérisé en ce que l'aminoacide basique est la lysine et/ou l'ornithine.

10. Agent diagnostique de la fonction rénale selon l'une des revendications 1 à 9, caractérisé en ce qu'il contient le système tampon à une concentration telle que la solution finale présente une osmolalité de 250 à 350 mosmol/kg de H₂O.

11. Agent diagnostique de la fonction rénale selon la revendication 10, caractérisé en ce que l'osmolalité est comprise entre 280 et 320 mosmol/kg de H₂O.

12. Agent diagnostique de la fonction rénale selon l'une des revendications 1 à 11, caractérisé en ce qu'il contient un fructane provenant de liliacées, d'amaryllidacées ou/et de graminées.

13. Agent diagnostique de la fonction rénale selon l'une des revendications 1 à 12, caractérisé en ce qu'il contient de la sinistrine comme fructane.

## Revendications (Revendications pour l'(les) Etat(s) contractant(s) suivant(s): ES)

1. Procédé de préparation d'un agent diagnostique de la fonction rénale pouvant être administré par voie parentérale, caractérisé en ce que l'on prépare un fructane sans pyrogène, bien soluble dans l'eau, par extraction de façon connue en soi, avec de l'eau, à partir d'éléments de plantes contenant du fructane, ultrafiltration de l'extrait aqueux sur une première membrane qui retient les hydrates de carbone et les protéines de masse molaire élevée mais qui laisse passer le fructane, et ultrafiltration du filtrat de la première ultrafiltration sur une seconde membrane qui retient le fructane, mais qui laisse passer les sels, les monosaccharides et les oligosaccharides inférieurs, et on ajoute à la solution de fructane ainsi obtenue un système tampon physiologiquement acceptable ayant un pH de 5,0 à 7,0 en une quantité telle que la solution finale est isotonique au sang.

2. Procédé selon la revendication 1, caractérisé en ce que l'on établit un pH compris entre 6,0 et 6,5.

3. Procédé selon la revendication 1 ou 2, caractérisé en ce que le système tampon est constitué d'un acide organique physiologiquement acceptable et d'un de ses sels de métaux alcalins et/ou de métaux alcalino-terreux.

4. Procédé selon l'une des revendications 1 à 3, caractérisé en ce que l'on utilise comme système tampon de l'acide lactique et du lactate de sodium.

5. Procédé selon l'une des revendications 1 à 3, caractérisé en ce que l'on utilise comme système tampon de l'acide acétique et de l'acétate de sodium.

6. Procédé selon l'une des revendications 1 et 2, caractérisé en ce que l'on utilise comme système tampon un mélange d'une base organique physiologiquement acceptable et d'un ou plusieurs de ses sels.

7. Procédé selon la revendication 6, caractérisé en ce que le système tampon est constitué par la triéthanolamine et son chlorhydrate.

8. Procédé selon l'une des revendications 1, 2 et 6, caractérisé en ce que le système tampon est constitué par un aminoacide basique et son chlorhydrate.

9. Procédé selon la revendication 8, caractérisé en ce que l'aminoacide basique est la lysine et/ou l'ornithine.

10. Procédé selon l'une des revendications 1 à 9, caractérisé en ce que l'on utilise le système tampon à une concentration telle que la solution finale présente une osmolalité de 250 à 350 mosmol/kg de H₂O.

11. Procédé selon la revendication 10, caractérisé en ce que l'osmolalité est comprise entre 280 et 320 mosmol/kg de H₂O.

12. Procédé selon l'une des revendications 1 à 11, caractérisé en ce que l'on utilise un fructane provenant de liliacées, d'amaryllidacées ou/et de graminées.

13. Procédé selon l'une des revendications 1 à 12, caractérisé en ce que l'on utilise de la sinistrine comme fructane.
